(19) Europäisches Patentamt
European Patent Office
Office européen des brevets

(11) EP 3 889 126 A1

(12) EUROPEAN PATENT APPLICATION
published in accordance with Art. 153(4) EPC

(43) Date of publication:
**06.10.2021 Bulletin 2021/40**

(21) Application number: **19890698.4**

(22) Date of filing: **26.11.2019**

(51) Int Cl.:
***C07C 41/08** (2006.01)* ***C07C 41/36** (2006.01)*
***C07C 41/42** (2006.01)* ***C07C 43/16** (2006.01)*
***C07B 61/00** (2006.01)* ***B01J 23/04** (2006.01)*

(86) International application number:
**PCT/JP2019/046072**

(87) International publication number:
**WO 2020/111031 (04.06.2020 Gazette 2020/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2018 JP 2018221036**

(71) Applicant: **Maruzen Petrochemical Co., Ltd.**
**Tokyo 104-8502 (JP)**

(72) Inventors:
- **NANIKI, Takashi**
  **Ichihara-shi, Chiba 290-8503 (JP)**
- **TAKAYAMA, Ayato**
  **Ichihara-shi, Chiba 290-8503 (JP)**
- **TSUCHIDA, Wataru**
  **Ichihara-shi, Chiba 290-8503 (JP)**
- **ITO, Jun**
  **Ichihara-shi, Chiba 290-8503 (JP)**
- **TAKAHASHI, Takashi**
  **Ichihara-shi, Chiba 290-8503 (JP)**
- **KANEMASA, Naoki**
  **Ichihara-shi, Chiba 290-8503 (JP)**

(74) Representative: **Hartz, Nikolai**
**Wächtershäuser & Hartz**
**Patentanwaltspartnerschaft mbB**
**Weinstrasse 8**
**80333 München (DE)**

# (54) METHOD FOR PRODUCING DIVINYL ETHER COMPOUND HAVING ALKYLENE SKELETON

(57) Provided is a new means capable of purifying a divinyl ether compound having an alkylene skeleton at a low energy and in a simplified manner.

For such object, a method for producing a divinyl ether compound, comprising following steps A and B: step A for reacting a compound represented by formula (1) with acetylene by using an alkali metal catalyst, and step B for purifying the divinyl ether compound represented by formula (2) without an extraction, which is obtained in the step A,

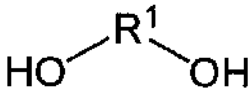

(1)

wherein $R^1$ represents an alkylene group having 4 to 20 carbon atoms, and

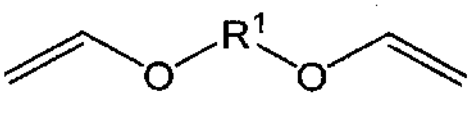

(2)

wherein $R^1$ has the same meaning as $R^1$ in formula (1).

EP 3 889 126 A1

## Description

Technical Field

The present invention relates to a method for producing a divinyl ether compound having an alkylene skeleton.

Background Art

A compound having two vinyl ether structures is called as a divinyl ether compound, and is used as a crosslinking component and a curing component of a raw material for a polymerization composition. Of this, the divinyl ether compound having an alkylene skeleton is expected to be applied to, for example, adhesives, paints, printing inks, and resist materials because of its excellent properties such as low toxicity, low irritation, and low shrinkage.
Leppe method is known as a method for producing a divinyl ether compound, in which an alcohol (diol) is reacted with acetylene in the presence of an alkali metal catalyst. For example, Patent Literature 1 discloses a method for separating and collecting diethylene glycol divinyl ether by reacting diethylene glycol and acetylene in the presence of diethylene glycol divinyl ether, proceeding with the reaction to the state where both diethylene glycol monovinyl ether and diethylene glycol divinyl ether are produced, and then performing extractive distillation and purification distillation.
In the production method described in Patent Literature 1, diethylene glycol divinyl ether can be collected with high purity. However, for such collection, it is necessary to conduct not only the purification distillation but also extractive distillation which requires a large amount of energy for the treatment. In addition, it is possible to reuse diethylene glycol divinyl ether as a raw material for a reaction, which is produced in a high proportion (about 30% by mass). However, for collecting the raw material, an excess amount of energy is required. Further, this method has not realized the production of a divinyl ether compound having an alkylene skeleton.
Further, as a method for synthesizing a divinyl ether compound having an alkylene skeleton and additionally purifying the same, the following method is known. Namely, a divinyl ether compound having an alkylene skeleton is produced from an alkanediol and acetylene in dimethyl sulfoxide as an aprotic polar solvent by the Leppe method, and then the resulting compound is extracted with a large amount of a hydrocarbon solvent (Patent Literature 2). However, when producing it in an industrial scale, this method has a concern of reduction in pot efficiency in a distillation step after the extraction, since a more energy is needed to the extractive treatment and further a large amount of the solvent is required for the extractive treatment.

Citation List

Patent Literature

Patent Literature 1: JP 2014-513048 A
Patent Literature 2: WO 2015/190376 A

Summary of Invention

Technical Problem

An object of the present invention is to provide a new means capable of purifying a divinyl ether compound having an alkylene skeleton at a low energy and in a simplified manner.

Solution to Problem

A problem of the present invention is solved by the following means <1> to <7>.

<1> A method for producing a divinyl ether compound (hereinafter, also referred to as the production method of the present invention, or the production method of the divinyl compound of the present invention), comprising following steps A and B:

step A for reacting a compound represented by formula (1) (hereinafter, also referred to as alkanediol (1)) with acetylene by using an alkali metal catalyst, and
step B for purifying the divinyl ether compound represented by formula (2) (hereinafter, also referred to as divinyl

ether compound (2)) without an extraction, which is obtained in the step A,

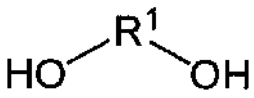
(1)

wherein $R^1$ represents an alkylene group having 4 to 20 carbon atoms, and

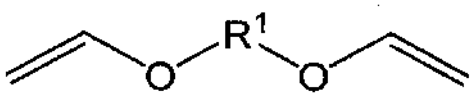
(2)

wherein $R^1$ has the same meaning as $R^1$ in formula (1).

<2> The production method according to <1>, wherein the compound represented by formula (1) is represented by formula (3), (4), or (5).

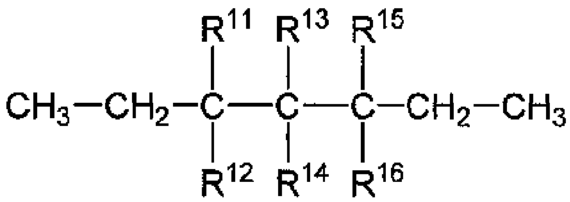
(3)

In formula (3), any two of $R^{11}$ to $R^{16}$ represent a hydroxy group or a hydroxymethyl group, and the others represent a hydrogen atom.

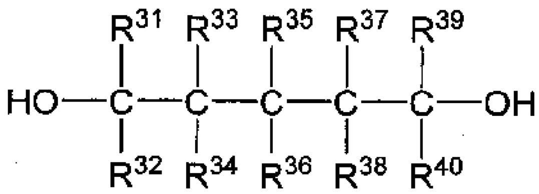
(4)

In formula (4), any one of $R^{31}$ to $R^{40}$ represents a methyl group, and the others represent a hydrogen atom.

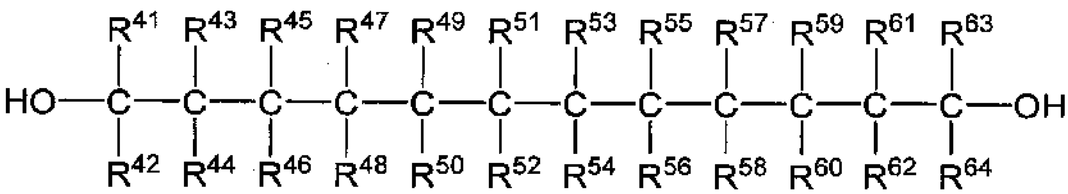
(5)

In formula (5), $R^{41}$ to $R^{64}$ represent a hydrogen atom or an alkyl group having 1 to 8 carbon atoms, provided that as for the combination of $R^{41}$ to $R^{64}$, any one of $R^{41}$ to $R^{64}$ is an alkyl group having 1 to 8 carbon atoms and the others are hydrogen atoms, or all of $R^{41}$ to $R^{64}$ are hydrogen atoms.

<3> The production method according to <1> or <2>, wherein the compound represented by formula (1) is at least one selected from the group consisting of 3-methyl-1,5-pentanediol, 2,4-diethyl-1,5-pentanediol, 2-butyl-2-ethyl-1,3-propanediol, 1,12-dodecanediol, and 1,12-octadecanediol.

<4> The production method according to any one of <1> to <3>, wherein the alkali metal catalyst is at least one selected from the group consisting of an alkali metal hydroxide and alkali metal carbonate.

<5> The production method according to any one of <1> to <4>, wherein the purification is conducted by at least one treatment selected from the group consisting of filtration, washing, drying, centrifugation, distillation, chromatography and membrane separation.
<6> The production method according to any one of <1> to <5>, wherein the reaction is performed in the absence of a solvent or in the presence of a grime solvent.
<7> The production method according to any one of <1> to <6>, wherein the reaction is performed in the presence of a grime solvent.

Advantageous Effects of Invention

According to the production method of the present invention, it is possible to purify a divinyl ether compound having an alkylene skeleton at a low energy and in a simplified manner.

Description of Embodiments

The production method of the divinyl ether compound of the present invention comprises following steps A and B: step A for reacting alkanediol (1) with acetylene by using an alkali metal catalyst, and step B for purifying the divinyl ether compound (2) obtained in the step A without an extraction.

In formulas (1) and (2), the alkylene group (alkanediyl group) represented by $R^1$ has carbon atoms of 4 to 20, preferably 6 to 18 from the viewpoint of the production rate, the reaction yield, the reduction in energy of the purification step and the simplification. In addition, the alkylene group may be linear or branched.

Specific examples of the alkylene group include butane-1,1-diyl group, butane-1,2-diyl group, butane-1,3-diyl group, butane-1,4-diyl group, pentane-1,1-diyl group, pentane-1,2-diyl group, pentane-1,3-diyl group, pentane-1,4-diyl group, pentane-1,5-diyl group, hexane-1,1-diyl group, hexane-1,2-diyl group, hexane-1,3-diyl group, hexane-1,4-diyl group, hexane-1,5-diyl group, hexane-1,6-diyl group, 3-methyl-pentane-1,5-diyl group, heptane-1,7-diyl group, octane-1,8-diyl group, nonane-1,9-diyl group, 2,4-diethyl-pentane-1, 5-diyl group, 2-butyl-2-ethyl-propane-1,3-diyl group, decane-1,10-diyl group, undecane-1,11-diyl group, dodecane-1,12-diyl group, tridecane-1,13-diyl group, tetradecane-1,14-diyl group, pentadecane-1,15-diyl group, hexadecane-1,16-diyl group, heptadecane-1,17-diyl group, octadecane-1,12-diyl group, and octadecane-1,18-diyl group.

(Step A)

In the step A, alkanediol (1) is reacted with acetylene by using an alkali metal catalyst.

Alkanediol (1) used in the present invention preferably has a boiling point of 50°C or more at normal pressure, and more preferably a boiling point of 100 to 450°C at normal pressure.

In addition, alkanediol (1) is preferably represented by the following formulas (3) to (5) from the viewpoints of the production rate, the reaction yield, the reduction in energy of the purification step and the simplification. In addition, examples of the alkanediol represented by formula (3) include those represented by formulas (3-1) to (3-3). The production method of the present invention can provide the corresponding divinyl ether compound with a high purity from the alkanediol having such a structure.

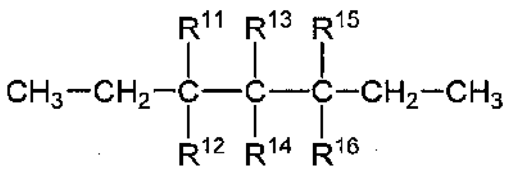

(3)

In formula (3), any two of $R^{11}$ to $R^{16}$ represent a hydroxy group or a hydroxymethyl group, and the others represent a hydrogen atom.

When representing a hydroxy group or a hydroxymethyl group, $R^{11}$ to $R^{16}$ may be the same or different from each other.

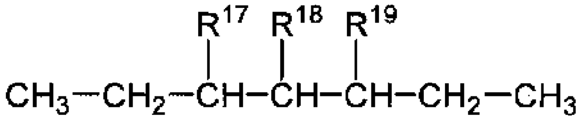

(3-1)

In formula (3-1), any two of $R^{17}$ to $R^{19}$ represent a hydroxy group or a hydroxymethyl group, and the others represent a hydrogen atom.

When representing a hydroxy group or a hydroxymethyl group, $R^{17}$ to $R^{19}$ may be the same or different from each other.

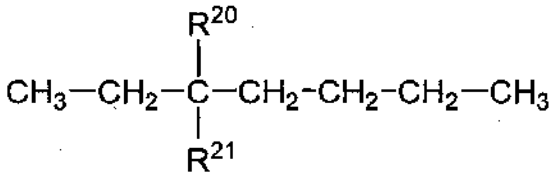

(3 − 2)

In formula (3-2), $R^{20}$ and $R^{21}$ each independently represent a hydroxy group or a hydroxymethyl group.

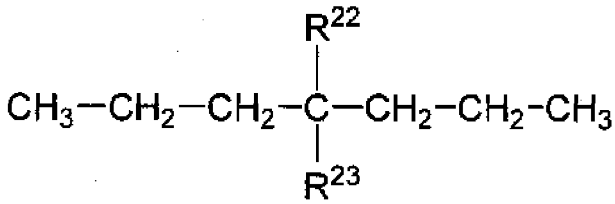

(3 − 3)

In formula (3-3), $R^{22}$ and $R^{23}$ each independently represent a hydroxy group or a hydroxymethyl group.

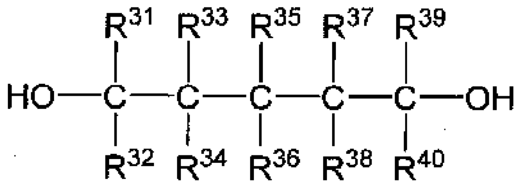

(4)

In formula (4), any one of $R^{31}$ to $R^{40}$ represents a methyl group, and the others represent a hydrogen atom.

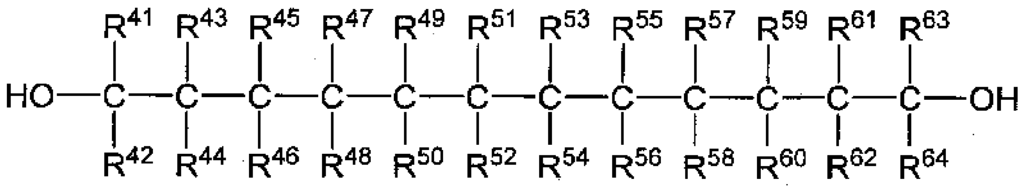

(5)

In formula (5), $R^{41}$ to $R^{64}$ represent a hydrogen atom or an alkyl group having 1 to 8 carbon atoms, provided that as for the combination of $R^{41}$ to $R^{64}$, any one of $R^{41}$ to $R^{64}$ is an alkyl group having 1 to 8 carbon atoms and the others are hydrogen atoms, or all of $R^{41}$ to $R^{64}$ are hydrogen atoms.

The alkyl groups represented by $R^{41}$ to $R^{64}$ may be linear or branched, and examples thereof include a methyl group, an ethyl group, a n-propyl group, an isopropyl group, a n-butyl group, an isobutyl group, a sec-butyl group, a tert-butyl group, a n-pentyl group, an isopentyl group, a neopentyl group, a n-hexyl group, an isohexyl group, a n-heptyl group, and a n-octyl group.

Specific examples of the alkanediol (1) include 1,2-butanediol, 1,3-butanediol, 1,4-butanediol, 2,3-butanediol, 2-methyl-1,3-propanediol, 1,2-pentanediol, 1,4-pentanediol, 1,5-pentanediol, 2,4-pentanediol, 2-methyl-1,3-butanediol, 3-methyl-1,3-butanediol, 2-methyl-2,3-butanediol, 2,2-dimethyl-1,3-propanediol, 1,2-hexanediol, 1,5-hexanediol, 1,6-hexanediol, 2,4-hexanediol, 2,5-hexanediol, 3-methyl-1,5-pentanediol, 4-methyl-2,3-pentanediol, hexylene glycol, 3,3-dimethyl-1,2-butanediol, 2,2-dimethyl-1,3-butanediol, pinacol, 2-ethyl-2-methyl-1,3-propanediol, 1,2-heptanediol, 1,4-heptanediol, 1,7-heptanediol, 3,3-heptanediol, 3,4-heptanediol, 3,5-heptanediol, 4,4-heptanediol, 5-methyl-2,4-hexanediol, 3,3-dimethyl-1,5-pentanediol, 2,4-dimethyl-2,4-pentanediol, 2,2-diethyl-1,3-propanediol, 2-methyl-2-propyl-1,3-propanediol, 1,2-octanediol, 1,8-octanediol, 4,5-octanediol, 3-methyl-2,4-heptanediol, 2-ethyl-1,2-hexanediol, 2-ethyl-1,3-hexanediol, 2-ethyl-1,4-hexanediol, 2,5-dimethyl-2,5-hexanediol, 2-propyl-1,2-pentanediol, 2,4,4-trimethyl-1,2-pentanediol, 2-propyl-1,3-pentanediol, 2-methyl-2-(1-methylpropyl)-1,3-propanediol, 2,2,4-trimethyl-1,3-pentanediol, 2,4,4-trimethyl-2,3-pentanediol, 1,2-nonanediol, 1,9-nonanediol, 2,4-diethyl-1,5-pentanediol, 2-ethyl-3-propyl-1,4-butanediol, 2-butyl-2-ethyl-1,3-propanediol, 1,2-decanediol, 1,10-decanediol, 2,7-dimethyl-2,7-octanediol, 3,6-dimethyl-3,6-octanediol, 2,3,4,5-tetramethyl-3,4-hexanediol,2,2-dibutyl-1,3-propanediol, 2,2-diisobutyl-1,3-propanediol, 1,2-dodecanediol, 1,12-dodecanediol, 5-ethyl-3-methyl-2,4-nonanediol, 7-ethyl-2-methyl-4,6-nonanediol, 2-butyl-1,3-octanediol, 2-methyl-2,3-dodecanediol, 2,2-diisoamyl-1,3-propanediol, 2-(4,4-dimethylpentyl)-2-propyl-1,3-propanediol, 1,2-tetradecanediol, 1,14-tetradecanediol, 2,2,9,9-tetramethyl-1,10-decandiol, 2-octyl-2-propyl-1,3-propanediol, 1,2-hexadecanediol, 1,16-hexadecanediol, 2-decyl-2-propyl-1,3-propanediol, 2-(2-methylpropyl)-2-nonyl-1,3-propanediol, 5,13-heptadecanediol, 2-dodecyl-2-ethyl-1,3-propanediol, 1,12-octadecanediol, 2,9-dimethyl-2,9-dipropyl-1,10-decanediol, 2-dodecyl-2-propyl-1,3-propanediol, 2,2-dioctyl-1,3-propanediol, and 8-ethyl-1,18-octadecanediol.

Of these, preferable is at least one selected from the group consisting of 3-methyl-1,5-pentanediol, 2,4-diethyl-1,5-pentanediol, 2-butyl-2-ethyl-1,3-propanediol, 1,12-dodecanediol, and 1,12-octadecanediol.

The alkali metal catalyst used in the present invention is not particularly limited, and conventionally known catalysts can be used. Examples thereof include alkali metal hydroxides such as potassium hydroxide and sodium hydroxide; and alkali metal carbonates such as potassium carbonate and sodium carbonate. The alkali metal catalyst may be used singly or in combination of two or more. Of these, alkali metal hydroxides are preferable from the viewpoint of the production rate, the reaction yield, the reduction in energy of the purification step and the simplification.

The amount of the alkali metal catalyst used is preferably in the range of 1 to 60 mol, and more preferably in the range of 5 to 45 mol with respect to 100 mol of alkanediol (1), from the viewpoints of the production rate, the reaction yield, the reduction in energy of the purification step, the simplification and production cost.

The supply pressure of acetylene in the production method of the present invention is not particularly limited, and from the viewpoints of safety and reaction progress, it is preferably 0.01 to 0.4 MPa, and more preferably 0.01 to 0.08 MPa, in terms of the gauge pressure.

The reaction temperature of the reaction between alkanediol (1) and acetylene is preferably in the range of 50 to 170°C, and more preferably in the range of 90 to 160°C, from the viewpoints of safety and reaction progress. When alkanediol (1) is pretreated with an alkali metal catalyst to be alkoxided prior to the supply of acetylene, the alkoxide reaction can also be performed at the same reaction temperature.

The reaction time of the reaction between alkanediol (1) and acetylene may be adjusted according to the type of alkanediol (1) for example, and is typically 1 to 72 hours, and preferably 1.5 to 48 hours.

In addition, the reaction of alkanediol (1) with acetylene can be performed by a batch method, a semi-continuous method, or a continuous method.

The reaction between alkanediol (1) and acetylene may be performed in the presence of a solvent or in the absence of a solvent. From the viewpoints of the production rate, the reaction yield, the reduction in energy of the purification step, the simplification, it is preferable that the reaction is conducted in the absence of a solvent or in the presence of a grime solvent. Since the reaction is conducted in the absence of a solvent or in the presence of a grime solvent, it is possible to obtain a high-purity of the divinyl ether compound (2) without an extraction at a low energy and in a simplified purification treatment. Note that the present inventors expect the reason why such effect is exerted when the grime solvent is used is that the grime solvent dissolves both alkanediol (1) and the divinyl ether compound (2).

The grime solvent means a solvent having a glycol ether structure. Examples thereof include ethylene glycol dimethyl ether, diethylene glycol dimethyl ether, triethylene glycol dimethyl ether, tetraethylene glycol dimethyl ether. Note that it may be used singly or in combination of two or more.

When the reaction is performed in the presence of a solvent, an amount of the solvent used is preferably 10 to 1000 parts by mass, more preferably 20 to 500 parts by mass with respect to 100 parts by mass of alkanediol (1).

When the reaction is performed in the absence of a solvent, an amount of alkanediol (1) to be used is preferably 85 to 99% by mass, more preferably 90 to 99% by mass with respect to a total amount of liquid phase (excluding the product) in a reaction system, from the viewpoint of the production cost and allowing the divinyl ether compound thus formed to a high purity.

In addition, when the reaction is performed in the absence of a solvent, a total proportion of alkanediol (1) and an alkali metal catalyst to be charged is preferably 95 to 100% by mass, more preferably 97.5 to 100% by mass with respect to a total amount of the components to be charged (excluding acetylene).

In addition, the order of contact of alkanediol (1), the acetylene, and the alkali metal catalyst is arbitrary. For example, it includes a method in which alkanediol (1), acetylene, and an alkali metal catalyst are supplied into the reactor and then the divinyl etherification reaction is conducted, or a method in which alkanediol (1) and an alkali metal catalyst are previously charged in the reactor, the temperature is raised to a predetermined temperature, and then the acetylene pressure in the reactor is increased to initiate the reaction. When alkanediol (1) and the alkali metal catalyst are previously charged in the reactor, the alkoxide reaction may proceed prior to the supply of acetylene. In addition, if the solvent is used, for example, the solvent may be supplied into the reactor when supplying alkanediol (1). The reaction pressure of the alkoxide reaction is preferably 1 to 101.3 kPa in terms of absolute pressure.

(Step B)

In the step B, the divinyl ether compound (2) obtained in the step A is purified without an extraction.

After completion of the reaction between alkanediol (1) and acetylene, the divinyl ether compound (2) obtained in the step A can be purified without an extraction at a low energy and in a simplified manner. As the method for purification, it includes, for example, filtration, washing, drying, centrifugation, distillation, chromatography and membrane separation. Of this, it may be used singly or in combination of two or more. For example, the targeted divinyl ether compound (2) may be isolated by separating a catalyst by filtration and then conducting distillation. The divinyl ether compound (2) is derived from alkanediol (1) and has a corresponding chemical structure.

As the filtration, it specifically includes filtration under normal pressure, filtration under pressure, filtration under reduced pressure or the like. In addition, a ventilation rate of filter such as filter paper or filter cloth is preferably 0.3 to 3.6 $cc/cm^2 \cdot sec$. Further, it is preferable to conduct the filtration at 25 to 150 °C.

The distillation is conducted by a distillation column. Example of distillation column to be used may be a packed column, a plate column, a bubble-cap column. A number of stages in the distillation column is preferably 1 to 50 stages, more preferably 1 to 30 stages, in terms of the theoretical stage. In addition, a reflux ratio preferably falls within 1 to 15.

The distillation may be conducted by any one of atmospheric distillation, pressure distillation or distillation under reduced pressure. Distillation under reduced pressure is preferable. A distillation pressure is preferably 0.01 to 20 kPa, more preferably 0.01 to 10 kPa. A distillation temperature is preferably 50 to 300°C. Further, a distillation mode may be a batch mode, a semi-continuous mode, or a continuous mode.

According to the production method of the present invention, it is possible to collect the divinyl ether compound (2) with the high purity at a low energy and in a simplified manner. In addition, it is possible to produce the divinyl ether compound (2) from alkanediol (1) and acetylene at a rapid production rate and a high reaction yield.

Example

Hereinafter, the present invention will be described in detail with reference to examples; however, the present invention is not limited to these examples. The measurement in the following examples was performed in accordance with the following measurement method.

Composition analysis of reaction solution

The composition of the reaction solution was analyzed by gas chromatography. The analysis conditions were as follows.

Equipment: Product name "GC-2010 Plus" (manufactured by Shimadzu Corporation)
Detector: FID
Column: DB-5 (30m, 0.25mmID, 1.0μm, manufactured by Agilent Technologies, Inc.)

• 1,12-Octadecane diol divinyl ether (ODDVE)

INJ temperature: 250°C
DET temperature: 250°C
Split ratio: 50
Column temperature: Holding for 3 min at 180°C → Heating up to 250°C at 10°C/min → Holding for 20 min at 250°C (30 min in total)

• 2,4-Diethyl-1,5-pentanediol divinyl ether (DEPDVE)

INJ temperature: 250°C
DET temperature: 300°C
Split ratio: 50
Column temperature: Heating up from 100°C to 160°C at 4°C/min → Holding for 10 min at 160°C → Heating up to 300°C at 20°C/min → Holding for 13 min at 300°C (45 min in total)

• 1,12-Dodecane diol divinyl ether (3DVE)

INJ temperature: 250°C
DET temperature: 270°C

Split ratio: 50
Column temperature: Holding for 3 min at 180°C → Heating up to 250°C at 5°C/min → Holding for 13 min at 250°C (30 min in total)

• 3-Methyl-1,5-pentanediol divinyl ether (MPDVE)

INJ temperature: 250°C
DET temperature: 300°C
Split ratio: 50
Column temperature: Holding 5 min at 100°C → Heating up to 280°C at 10°C/min → Holding 7 min at 280°C (30 min in total)

• 2-Butyl-2-ethyl-1,3-propanediol divinyl ether (BEPDVE)

INJ temperature: 200°C
DET temperature: 300°C
Split ratio: 70
Column temperature: Heating up from 100°C to 300°C at 8°C/min → Holding for 5 min at 300°C (30 min in total)

Potassium concentration measurement

The potassium concentration of the reaction solution was measured by the following equipment.

Equipment: Product name "Automatic titrator COM-1700A" (manufactured by HIRANUMA SANGYO Co., Ltd.)
Titrant: 0.2 mol/L hydrochloric acid and ethanol

Measurement of moisture content of distillate

The moisture content of the distillate was measured by using the following equipment.

Equipment: Product name "automatic moisture measuring equipment AQV-300" (manufactured by HIRANUMA SANGYO Co., Ltd.)
Titrant: HYDRANAL Composite 5K (manufactured by HAYASHI PURE CHEMICAL IND., LTD.)
Titration solvent: Karl Fischer reagent HAYASHI-solvent CE (manufactured by HAYASHI PURE CHEMICAL IND., LTD.)

Measurement of reaction rate

The reaction rate was calculated by the following formula.

$$r1 = M/\{(t1)\times V\}$$

r1: Reaction rate (g/L·hr)
M: Divinyl ether production amount (g)
t1: Reaction time (hr)
V: Reaction volume (L)

Measurement of production rate in reaction step

The production rate in the reaction step was calculated by the following formula.

$$r2 = M/\{(t1+t2)\times V\}$$

r2: Production rate in reaction step (g/L·hr)
M: Divinyl ether production amount (g)
t1: Reaction time (hr)

t2: Time of preparing potassium alkoxide (hr)
V: Reaction volume (L)

Measurement of distillation energy

The reaction solution after the completion of reaction was subjected to a composition analysis by chromatography. From the composition of the reaction solution and the evaporative latent heat of the substance contained therein, the required distillation energy per 1 kg of product was calculated.
Similarly, as for the distillation energy at the collection of solvent in an amount of solvent used per 1 kg of product, it was calculated from the composition of the lower layer of extraction solution and the evaporative latent heat of the substance contained therein by subjecting the lower layer of extraction solution to the composition analysis by chromatography.

Example 1: Synthesis of 1,12-octadecanediol divinyl ether (ODDVE)

0.18 kg (3.3 mol) of potassium hydroxide (manufactured by Nippon Soda Co., Ltd.) and 3.50 kg (12.2 mol) of 1,12-octadecanediol (manufactured by KOKURA SYNTHETIC INDUSTRIES, LTD.) that had been previously dissolved by heating were charged in a pressure-resistant reaction vessel made of SUS316, having a capacity of 10L, and equipped with a stirrer, pressure gauge, thermometer, gas introduction pipe, gas purge line, decompression line, and liquid sampling line, and nitrogen was purged inside the vessel.
After nitrogen purge, the temperature inside the vessel was raised to 150°C and stirring was performed at 250 rpm. The pressure inside the vessel was gradually reduced to 3 kPaA (A indicates absolute pressure), and bubbling with 0.1 NL/min nitrogen was performed for 3 hr from the liquid sampling line. Through the above operation, 0.06 kg of a distillate including water as the main component was taken out. Thus, potassium alkoxide derived from 1,12-octadecanediol was obtained.
Then, the inside of the vessel was purged with acetylene while stirring at 420 rpm. Acetylene was continuously supplied, the inside of the vessel was kept at 0.03 MPaG (G indicates gauge pressure) and 150°C, and the reaction was performed for 11.5 hr. As a result of gas chromatography analysis of the reaction solution, the conversion rate was 99% or more, and the selectivity was 97%. The reaction rate in this case was calculated to be 61 g/L·hr, and the production rate in reaction step was calculated to be 49 g/L·hr.
This reaction solution was subjected to filtration under pressure at 120°C and a nitrogen pressure of 0.1 MPaG by using a filter cloth (air permeability: 0.6 to 1.8 $cc/cm^2 \cdot sec$, made of polyphenylene sulfide resin) to separate a catalyst. The potassium concentration in the filtrate was 0.4% by mass. Moreover, simple distillation of the filtrate was performed under a reduced pressure of 0.02 kPaA to obtain 3.37 kg (10.0 mol) of 1,12-octadecanediol divinyl ether (hereinafter referred to as ODDVE). The obtained ODDVE had a purity of 99% or more and a yield of 81%. In addition, the required energy at the distillation was calculated as 0.2 MJ per 1 kg of ODDVE. The results are shown in Table 1.

Example 2: Synthesis of 2,4-diethyl-1,5-pentanediol divinyl ether (DEPDVE)

Into the same reaction vessel as in Example 1, 3.80 kg (23.7 mol) of 2,4-diethyl-1,5-pentanediol (manufactured by KH Neochem Co., Ltd.) and 0.29 kg (5.2 mol) of potassium hydroxide were charged, and nitrogen was purged inside the vessel.
After nitrogen purge, the temperature inside the vessel was raised to 150°C and stirring was performed at 250 rpm. Bubbling with 1 NL/min nitrogen was performed for 3 hr from the liquid sampling line. Through the above operation, 0.09 kg of a distillate including water as the main component was taken out. Thus, potassium alkoxide derived from 2,4-diethyl-1,5-pentanediol was obtained.
Then, the temperature in the vessel was raised to 155°C, and the inside of the vessel was purged with acetylene while stirring at 420 rpm. Acetylene was continuously supplied, the inside of the vessel was kept at 0.03 MPaG and 155°C, and the reaction was performed for 11.5 hr. As a result of gas chromatography analysis of the reaction solution, the conversion rate was 99% or more, and the selectivity was 97%. The reaction rate in this case was calculated to be 60 g/L·hr, and the production rate in reaction step was calculated to be 47 g/L·hr.
This reaction solution was subjected to filtration under pressure at 120°C and a nitrogen pressure of 0.1 MPaG by using the same filter cloth as in Example 1 to separate a catalyst. The potassium concentration in the filtrate was 0.2% by mass. Moreover, fine distillation of the filtrate was performed by using a distillation column manufactured by Kiriyama Glass Works Co. with 5 theoretical stages under the conditions of a pressure of 1.3 kPaA and a reflux ratio of 1 to obtain 3.50 kg (16.5 mol) of 2,4-diethyl-1,5-pentanediol divinyl ether (hereinafter referred to as DEPDVE). The obtained DEPDVE had a purity of 99% or more and a yield of 70%. In addition, the required energy at the distillation was calculated as 0.6 MJ per 1 kg of DEPDVE. The results are shown in Table 1.

Example 3: Synthesis of 1,12-dodecanediol divinyl ether (3DVE)

Into the same reaction vessel as in Example 1, 0.23 kg (4.1 mol) of potassium hydroxide and 3.96 kg (19.6 mol) of 1,12-dodecanediol (manufactured by FUJIFILM Wako Chemical Corporation) that had previously dissolved by heating were charged, and nitrogen was purged inside the vessel.
After nitrogen purge, the temperature inside the vessel was raised to 135°C and stirring was performed at 250 rpm. The pressure inside the vessel was gradually reduced to 20 kPaA, and bubbling with 0.1 NL/min nitrogen was performed for 3 hr from the liquid sampling line. Through the above operation, 0.09 kg of a distillate including water as the main component was taken out. Thus, potassium alkoxide derived from 1,12-dodecanediol was obtained.
Then, the temperature in the vessel was raised to 155°C, and the inside of the vessel was purged with acetylene while stirring at 420 rpm. Acetylene was continuously supplied, the inside of the vessel was kept at 0.03 MPaG and 155°C, and the reaction was performed for 17 hr. As a result of gas chromatography analysis of the reaction solution, the conversion rate was 99% or more, and the selectivity was 94%. The reaction rate in this case was calculated to be 52 g/L·hr, and the production rate in reaction step was calculated to be 44 g/L·hr.
This reaction solution was subjected to filtration under pressure at 120°C and a nitrogen pressure of 0.1 MPaG by using the same filter cloth as in Example 1 to separate a catalyst. The potassium concentration in the filtrate was 0.2% by mass. Moreover, simple distillation of the filtrate was performed under a reduced pressure of 0.06 kPaA to obtain 3.34 kg (13.1 mol) of 1,12-dodecanediol divinyl ether (hereinafter referred to as 3DVE). The obtained 3DVE had a purity of 99% or more, a melting point of 27°C and a yield of 67%. In addition, the required energy at the distillation was calculated as 0.3 MJ per 1 kg of 3DVE. The results are shown in Table 1.

Example 4: Synthesis of 3-methyl-1,5-pentanediol divinyl ether (MPDVE)

Into the same reaction vessel as in Example 1, 3.40 kg (28.8 mol) of 3-methyl-1,5-pentanediol (manufactured by FUJIFILM Wako Chemical Corporation) and 0.34 kg (6.0 mol) of potassium hydroxide were charged, and nitrogen was purged inside the vessel.
After nitrogen purge, the temperature inside the vessel was raised to 110°C and stirring was performed at 250 rpm. The pressure inside the vessel was gradually reduced to 5.5 kPaA, and bubbling with 0.1 NL/min nitrogen was performed for 3 hr from the liquid sampling line. Through the above operation, 0.11 kg of a distillate including water as the main component was taken out. Thus, potassium alkoxide derived from 3-methyl-1,5-pentanediol was obtained.
Then, the temperature in the vessel was raised to 155°C, and the inside of the vessel was purged with acetylene while stirring at 420 rpm. Acetylene was continuously supplied, the inside of the vessel was kept at 0.03 MPaG and 155°C, and the reaction was performed for 10 hr. Then, the temperature inside the vessel was lowered to 135°C, and the reaction was further performed for 3.5 hr. As a result of gas chromatography analysis of the reaction solution, the conversion rate was 99% or more, and the selectivity was 91%. The reaction rate in this case was calculated to be 62 g/L·hr, and the production rate in reaction step was calculated to be 51 g/L·hr.
This reaction solution was subjected to filtration under pressure at 120°C and a nitrogen pressure of 0.1 MPaG by using the same filter cloth as in Example 1 to separate a catalyst. The potassium concentration in the filtrate was 0.2% by mass. Moreover, simple distillation of the filtrate was performed under a reduced pressure of 1.3 kPaA to obtain 3.41 kg (20.0 mol) of 3-methyl-1,5-pentanediol divinyl ether (hereinafter referred to as MPDVE). The obtained MPDVE had a purity of 99% or more and a yield of 70%. In addition, the required energy at the distillation was calculated as 0.3 MJ per 1 kg of MPDVE. The results are shown in Table 1.

Example 5: Synthesis of 2-butyl-2-ethyl-1,3-propanediol divinyl ether (BEPDVE)

Into the same reaction vessel as in Example 1, 0.32 kg (5.7 mol) of potassium hydroxide, 2.20 kg (13.7 mol) of 2-butyl-2-ethyl-1,3-propanediol (KH Neochem Co., Ltd.) that had previously dissolved by heating, and 2.20 kg (9.9 mol) of tetraethylene glycol dimethyl ether (KISHIDA CHEMICAL Co., Ltd.) were charged, and nitrogen was purged inside the vessel.
After nitrogen purge, the temperature inside the vessel was raised to 90°C and stirring was performed at 250 rpm. The pressure inside the vessel was gradually reduced to 3 kPaA, and bubbling with 0.1 NL/min nitrogen was performed for 3 hr from the liquid sampling line. Through the above operation, 0.10 kg of a distillate including water as the main component was taken out. Thus, potassium alkoxide derived from 2-butyl-2-ethyl-1,3-propanediol was obtained.
After that, the temperature inside the vessel was raised to 110°C, and then the inside of the vessel was purged with acetylene, while stirring the inside of the vessel at 420 rpm. Acetylene was continuously supplied, the inside of the vessel was kept at 0.03 MPaG and 110°C, and the reaction was performed for 10.5 hr. As a result of gas chromatography analysis of the reaction solution, the conversion rate was 99% or more, and the selectivity was 80%.
This reaction solution was subjected to filtration under pressure at a room temperature and a nitrogen pressure

of 0.1 MPaG by using the same filter cloth as that of Example 1, to separate a catalyst. The potassium concentration in the filtrate was 0.1% by mass. Moreover, fine distillation of the filtrate was performed by using a distillation column manufactured by Kiriyama Glass Works Co. with 15 theoretical stages. Under the conditions of a pressure of 1.3 kPaA and a reflux ratio of 20, the first fraction was separated, and then the distillation was performed at a reflux ratio of 1, to obtain 1.95 kg (9.1 mol) of 2-butyl-2-ethyl-1,3-propanediol divinyl ether (hereinafter referred to as BEPDVE). The obtained BEPDVE had a purity of 99% or more and a yield of 67%. In addition, the required energy at the distillation was calculated as 2.0 MJ per 1 kg of BEPDVE. Further, the required energy when continuing the distillation at a reflux ratio of 1 and collecting 1.5 kg of tetraethylene glycol dimethyl ether corresponding to 70% with respect to the charged amount, was calculated as 0.4 MJ per 1 kg of BEPDVE. The results are shown in Table 1.

Comparative Example 1: Synthesis of 2,4-diethyl-1,5-pentanediol divinyl ether (DEPDVE)

Into the same reaction vessel as in Example 1, 1.00 kg (6.3 mol) of 2,4-diethyl-1,5-pentanediol, 0.10 kg (1.8 mol) of potassium hydroxide, and 4.00 kg of dimethyl sulfoxide were charged, and nitrogen was purged inside the vessel.

While stirring the inside of the vessel at 420 rpm, the temperature was raised to 80°C, and then the inside of the vessel was purged with acetylene. Acetylene was continuously supplied, the pressure inside the vessel was kept at 0.03 MPaG, and the reaction was performed for 7 hr at a temperature of 80°C inside the vessel. As a result of gas chromatography analysis of the reaction solution, the conversion rate was 99% or more, and the selectivity was 90%. The reaction rate in this case was calculated to be 32 g/L·hr, and the production rate in reaction step was calculated to be 32 g/L·hr.

This reaction solution was transferred to a 20 L plastic container, 5.20 kg of hexane was added, and the mixture was shaken and allowed to stand. After standing, the upper layer was removed and concentrated under reduced pressure. Moreover, fine distillation of the concentrated solution was performed by using a distillation column manufactured by Kiriyama Glass Works Co. with 10 theoretical stages. 0.87 kg (4.1 mol) of DEPDVE was obtained under the conditions of a pressure of 1.3 kPaA and a reflux ratio of 10. The obtained DEPDVE had a purity of 99% or more and a yield of 65%. In addition, the required energy at the concentration and distillation was calculated as 7.6 MJ per 1 kg of DEPDVE. Further, the required energy when collecting 2.8 kg of dimethyl sulfoxide corresponding to 70% with respect to the charged amount, from the lower layer of extraction solution by the simple distillation, was calculated as 1.8 MJ per 1 kg of DEPDVE. The results are shown in Table 1.

Comparative Example 2: Synthesis of 2-butyl-2-ethyl-1,3-propanediol divinyl ether (BEPDVE)

Into the same reaction vessel as in Example 1, 1.00 kg (6.3 mol) of 2-butyl-2-ethyl-1,3-propanediol that had previously dissolved by heating, 0.20 kg (3.6mol) of potassium hydroxide, and 4.00 kg of dimethyl sulfoxide were charged, and nitrogen was purged inside the vessel.

While stirring the inside of the vessel at 420 rpm, the temperature was raised to 80°C, and then the inside of the vessel was purged with acetylene. Acetylene was continuously supplied, the pressure inside the vessel was kept at 0.03 MPaG, and the reaction was performed for 7 hr at a temperature of 80°C inside the vessel. As a result of gas chromatography analysis of the reaction solution, the conversion rate was 99% or more, and the selectivity was 92%.

This reaction solution was transferred to a 20 L plastic container, 5.34 kg of heptane was added, and the mixture was shaken and allowed to stand. After standing, the upper layer was removed and concentrated under reduced pressure. Moreover, fine distillation of the concentrated solution was performed by using a distillation column manufactured by Kiriyama Glass Works Co. with 10 theoretical stages. 0.94 kg (4.4 mol) of BEPDVE was obtained under the conditions of a pressure of 1.3 kPaA and a reflux ratio of 10. The obtained BEPDVE had a purity of 99% or more and a yield of 70%. In addition, the required energy at the concentration and distillation was calculated as 6.9 MJ per 1 kg of BEPDVE. Further, the required energy when collecting 2.8 kg of dimethyl sulfoxide corresponding to 70% with respect to the charged amount, from the lower layer of extraction solution by the simple distillation, was calculated as 1.6 MJ per 1 kg of BEPDVE. The results are shown in Table 1.

[Table 1]

| | Targeted compound | Reaction solvent | Catalyst separation | Distillation | Yield (%) | Distillation energy | | |
|---|---|---|---|---|---|---|---|---|
| | | | | | | Product (MJ/kg) | Solvent collection (MJ/kg) | Total (MJ/kg) |
| Example 1 | ODDVE | - | Filtration | Simple distillation | 81 | 0.2 | - | 0.2 |

(continued)

| | Targeted compound | Reaction solvent | Catalyst separation | Distillation | Yield (%) | Distillation energy | | |
|---|---|---|---|---|---|---|---|---|
| | | | | | | Product (MJ/kg) | Solvent collection (MJ/kg) | Total (MJ/kg) |
| Example 2 | DEPDVE | - | Filtration | 5 stages | 70 | 0.6 | - | 0.6 |
| Example 3 | 3DVE | - | Filtration | Simple distillation | 67 | 0.3 | - | 0.3 |
| Example 4 | MPDVE | - | Filtration | Simple distillation | 70 | 0.3 | - | 0.3 |
| Example 5 | BEPDVE | GL4 | Filtration | 15 stages | 67 | 2.0 | 0.4 | 2.4 |
| Comparative Example 1 | ODDVE | DMSO | Extraction | 10 stages | 65 | 7.6 | 1.8 | 9.4 |
| Comparative Example 2 | DEPDVE | DMSO | Extraction | 10 stages | 70 | 6.9 | 1.6 | 8.5 |

The symbols in the table indicate the following.

ODDVE: 1,12-Octadecanediol divinyl ether
DEPDVE: 2,4-Diethyl-1,5-pentanediol divinyl ether
3DVE: 1,12-Dodecanediol divinyl ether
MPDVE: 3-Methyl-1,5-pentanediol divinyl ether
BEPDVE: 2-butyl-2-ethyl-1,3-propanediol divinyl ether
GL4: tetraethylene glycol dimethyl ether
DMSO: Dimethyl sulfoxide

As shown in Table 1, when alkanediol (1) was reacted with acetylene in the absence of a solvent (Examples 1 to 4), or when performing such reaction in the presence of a grime solvent (Example 5), a divinyl ether compound having an alkylene skeleton could be purified without an extraction at a low energy and in a simplified manner

**Claims**

**1.** A method for producing a divinyl ether compound, comprising following steps A and B:

step A for reacting a compound represented by formula (1) with acetylene by using an alkali metal catalyst, and step B for purifying the divinyl ether compound represented by formula (2) without an extraction, which is obtained in the step A,

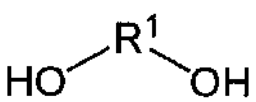
(1)

wherein $R^1$ represents an alkylene group having 4 to 20 carbon atoms, and

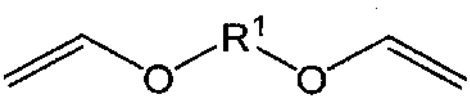
(2)

wherein $R^1$ has the same meaning as $R^1$ in formula (1).

**2.** The production method according to claim 1, wherein the compound represented by formula (1) is represented by formula (3), (4), or (5),

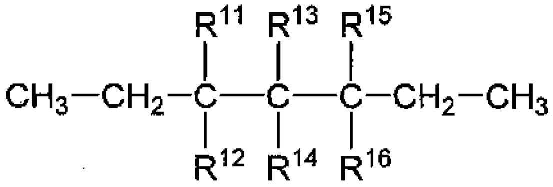

(3)

wherein any two of $R^{11}$ to $R^{16}$ represent a hydroxy group or a hydroxymethyl group and the others represent a hydrogen atom,

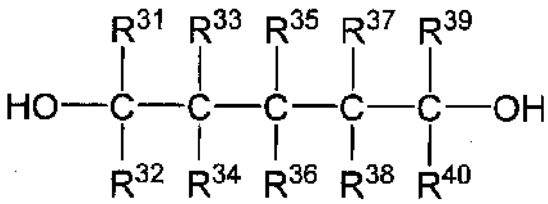

(4)

wherein any one of $R^{31}$ to $R^{40}$ represents a methyl group and the others represent a hydrogen atom, and

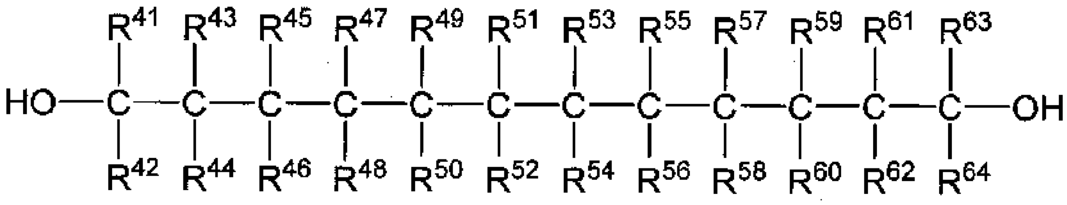

(5)

wherein $R^{41}$ to $R^{64}$ represent a hydrogen atom or an alkyl group having 1 to 8 carbon atoms, provided that as for a combination of $R^{41}$ to $R^{64}$, any one of $R^{41}$ to $R^{64}$ is an alkyl group having 1 to 8 carbon atoms and the others are hydrogen atoms, or all of $R^{41}$ to $R^{64}$ are hydrogen atoms.

**3.** The production method according to claim 1 or 2, wherein the compound represented by formula (1) is at least one selected from the group consisting of 3-methyl-1,5-pentanediol, 2,4-diethyl-1,5-pentanediol, 2-butyl-2-ethyl-1,3-propanediol, 1,12-dodecanediol, and 1,12-octadecanediol.

**4.** The production method according to any one of claims 1 to 3, wherein the alkali metal catalyst is at least one selected from the group consisting of an alkali metal hydroxide and alkali metal carbonate.

**5.** The production method according to any one of claims 1 to 4, wherein the purification is conducted by at least one treatment selected from the group consisting of filtration, washing, drying, centrifugation, distillation, chromatography and membrane separation.

**6.** The production method according to any one of claims 1 to 5, wherein the reaction is performed in the absence of a solvent or in the presence of a grime solvent.

**7.** The production method according to any one of claims 1 to 6, wherein the reaction is performed in the presence of a grime solvent.

**INTERNATIONAL SEARCH REPORT**

International application No.
PCT/JP2019/046072

A. CLASSIFICATION OF SUBJECT MATTER
C07C 41/08(2006.01)i; C07C 41/36(2006.01)i; C07C 41/42(2006.01)i; C07C 43/16(2006.01)i; C07B 61/00(2006.01)n; B01J 23/04(2006.01)i
FI: C07C41/08; C07C43/16; C07C41/36; C07C41/42; B01J23/04 Z; C07B61/00 300
According to International Patent Classification (IPC) or to both national classification and IPC

B. FIELDS SEARCHED
Minimum documentation searched (classification system followed by classification symbols)
C07C41/08; C07C41/36; C07C41/42; C07C43/16; C07B61/00; B01J23/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN), CASREACT (STN)

C. DOCUMENTS CONSIDERED TO BE RELEVANT

| Category* | Citation of document, with indication, where appropriate, of the relevant passages | Relevant to claim No. |
|---|---|---|
| X | CRIVELLO, James V., MCGRATH, Thomas M., "Photo initiated alternating copolymerization of dialkyl maleates and fumarates with vinyl ethers", Journal of Polymer Science, Part A: Polymer Chemistry, 07 September 2010, vol. 48, no. 21, pp. 4726-4736, page 4727, right column, General Preparation of Difunctional Vinyl Ethers | 1-7 |
| X | DE 19924159 Al (BASF AG) 30.11.2000 (2000-11-30) Beispiele 1, 7-14, Patentanspruche | 1-7 |
| X | JP 5-279284 A (NIPPON CARBIDE INDUSTRIES CO., INC.) 26.10.1993 (1993-10-26) paragraphs [0017]-[0019], [0022]-[0029] | 1-7 |
| X | JP 4-500520 A (EXFLUOR RESEARCH CORPORATION) 30.01.1992 (1992-01-30) example 39 | 1-7 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 February 2020 (19.02.2020) | 03 March 2019 (03.03.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/046072

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | CN 102173982 A (NANJING FORESTRY UNIVERSITY) 07.09.2011 (2011-09-07) claims, paragraphs [0017], [0019]-[0025], [0028] | 1-7<br>1-7 |
| X | JP 8-225479 A (BASF AG.) 03.09.1996 (1996-09-03) claims, paragraphs [0014], [0021], [0030]-[0035] | 1-7 |
| Y | WO 2015/190376 A1 (MARUZEN PETROCHEMICAL CO., LTD.) 17.12.2015 (2015-12-17) claim 2, paragraphs [0026], [0040]-[0062] | 1-7 |

Form PCT/ISA/210 (continuation of second sheet) (January 2015)

**INTERNATIONAL SEARCH REPORT**
Information on patent family members

International application No.
PCT/JP2019/046072

| Patent Documents referred in the Report | Publication Date | Patent Family | Publication Date |
|---|---|---|---|
| DE 19924159 A1 | 30 Nov. 2000 | (Family: none) | |
| JP 5-279284 A | 26 Oct. 1993 | (Family: none) | |
| JP 4-500520 A | 30 Jan. 1992 | US 5093432 A<br>example 39<br>WO 1990/003353 A1<br>EP 441807 A1<br>KR 10-1995-0034940 A | |
| CN 102173982 A | 07 Sep. 2011 | (Family: none) | |
| JP 8-225479 A | 03 Sep. 1996 | US 5723685 A<br>claims, column 2, lines 15-19, 47-53, examples<br>EP 709359 A1 | |
| WO 2015/190376 A1 | 17 Dec. 2015 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014513048 A
- WO 2015190376 A